Europäisches Patentamt

European Patent Office

Office européen des brevets

(11) Publication number: **0 253 193 B1**

# EUROPEAN PATENT SPECIFICATION

(45) Date of publication of patent specification: **28.10.92**  (51) Int. Cl.⁵: **C12N 15/00**, C12Q 1/68

(21) Application number: **87109357.1**

(22) Date of filing: **30.06.87**

(54) **Method for mutagenesis by oligonucleotide-directed repair of a strand break.**

(30) Priority: **08.07.86 US 883242**

(43) Date of publication of application:
**20.01.88 Bulletin 88/03**

(45) Publication of the grant of the patent:
**28.10.92 Bulletin 92/44**

(84) Designated Contracting States:
**BE DE FR IT**

(56) References cited:
**EP-A- 0 139 501**
**EP-A- 0 155 188**

(73) Proprietor: **ABBOTT LABORATORIES**
**14th Street and Sheridan Road**
**North Chicago, Illinois 60064(US)**

(72) Inventor: **Mandecki, Wlodzimierz**
**131 Annapolis Drive**
**Vernon Hills Illinois 60061(US)**

(74) Representative: **Modiano, Guido et al**
**c/o Modiano & Associati S.r.l. Via Meravigli,**
**16**
**I-20123 Milano(IT)**

## Description

The present invention relates in general to methods for site-specific mutagenesis and for repair of DNA strand breaks. In particular, the present invention relates to methods for site-specific mutagenesis or DNA break repair wherein an oligonucleotide bearing a desired mutation is employed.

Site-specific mutagenesis is a term which is applied to a large set of in vitro methods [reviewed in Botstein et al., Science, 229, 1193-1201 (1985)] which set is generally described as including two classes of procedures: mutagenesis by primer extension and mutagenesis by a gene fragment replacement. In vivo, techniques involving homologous recombination allow targetting of specific sites for mutations.

Primer extension mutagenesis involves hybridization of a mutation-bearing oligonucleotide primer with single-stranded DNA which is the target of mutagenesis. The target DNA serves as a template for the in vitro enzymatic extension of the primer into a complete second strand. The resulting heterologous double-stranded DNA is introduced by transformation into E. coli. A fraction of clones resulting from this transformation carry the mutation.

Gene fragment replacement mutagenesis involves the restriction enzyme digestion in vitro of a double-stranded vector on the outside of a targeted region. Double-stranded synthetic DNA containing a mutation is ligated into the the vector at the restriction site.

The primer extension method was initially applied to the site-specific mutagenesis of the single-stranded bacteriophage φ174 [Hutchinson, J. Biol. Chem., 253, 6551 (1978); Razin et al., Proc. Natl. Acad. Sci. (USA), 75 4268 (1978)]. A number of protocols allow application of the method to any gene clone onto a circular vector. The requirement for single-stranded target DNA may be satisfied by: (i) the use of an M13 single-stranded vector [Zoller et al., Nucleic Acids Res., 10, 6487-6500 (1982)]; (ii) the use of a plasmid vector with the origin of replication from M13, which leads to packaging of single-stranded plasmid into virus particles after superinfection with M13 [Dente et al., Nucleic Acids Res., 11, 1645 (1983); Zugursky et al., Gene, 27, 183 (1984); and Levinson et al., J. Mol. Appl. Genet., 2, 507 (1984)]; (iii) the enzymatic degradation of a part of one strand of a duplex plasmid with an exonuclease [Wallace et al., Science, 209, 1396 (1980)]; (iv) the creation of a single-stranded gap by forming a heteroduplex molecule [Kramer et al., Nucleic Acids Res., 12, 9441 (1984)]; or (v) the direct annealing of the oligonucleotide to duplex DNA to form a D-loop [Schold et al., DNA, 3, (1984)].

The mutagenesis efficiency of the primer extension methods may be increased by using two primers to initiate DNA synthesis at two sites [Norris et al., Nucleic Acids Res., 11, 5103 (1983); and Zoller et al., DNA, 3, 479 (1984)], or by using a DNA template containing several uracil residues in place of thymine [Kunkel, Proc. Natl. Acad. Sci. (USA)), 82, 488-492 (1985)].

Synthetic DNA to be used for mutagenesis by gene fragment replacement may consist of two annealed oligonucleotides having ends which match ends created by restriction enzymes in vector DNA. Alternatively, mutants may be obtained via the construction of longer gene fragments composed of four or more oligonucleotides, or even whole synthetic genes, [Caruthers et al., "Promoters: Structure and Function", eds. Rodriguez et al., Praeger Scientific, New York, pp. 432-451 (1982)]. Specialized applications of the method include: (i) linker scanner mutagenesis for introducing clusters of point mutations at discrete locations [McKnight et al., Science, 217, 316-324 (1982)]; (ii) two codon insertion mutagenesis with the use of selectable marker [Barany, Proc. Natl. Acad. Sci. (USA), 82, 4202-4206 (1982)]; and (iii) "bandaid" mutagenesis which relies on ligating a single oligonucleotide into a double-stranded gap created by cleavage with two restriction endonucleases which form a single-stranded 3′ end and a single-stranded 5′ end respectively complementary to either end of the oligonucleotide [Mural et al., DNA, 5, 84 (1986)].

The above methods are universal and permit introduction of defined point mutations, insertions and delections in virtually any desired location on a cloned DNA. Therefore, site-directed mutagenesis is widely used to probe the structure-function relationship in DNA, RNA and protein, to study the interaction of these biological macromolecules and to elucidate pathways of genetic regulation. The broad applicability of site-directed mutagenesis warrants interest in novel mutagenesis methods, especially in methods which reduce the time and effort currently required to obtain a mutant.

Although the phenomena of primer extension and gene fragment replacement are well explained, the phenomenon of DNA double-strand break repair in E. coli is not well understood. Plasmid DNA linearized at a unique restriction site transforms E. coli, although with much lower efficiency than the corresponding covalently closed circular DNA form [Thompson et al., Mol. Gen. Genet., 169, 49-57 (1979); and Conley et al., Mol. Gen. Genet., 194, 211-218 (1984)]. Although transformants containing plasmids bearing deletions or rearrangements are frequent, transformants carrying the native plasmid molecules are also obtained. Simple intracelluloar reannealing and ligation of the two cohesive termini of a linear molecule is thought not to be the predominant mechanism of plasmid establishment, since transformation with linearized plasmid DNA is reduced 10 to 40-fold in recA, recBC, or recF backgrounds [Conley et al., Mol. Gen. Genet., 194, 211-218 (1984)].

In a variation of a process called homologous recombination, linearized chimeric plasmid DNA may transform cells containing a resident plasmid which is homologous with a portion of the chimeric plasmid DNA in the process called "marker rescue transformation". Marker rescue transformation is recE-dependent, requires that the chimeric and resident plasmids have homologous sequences and that homologous sequences flank any nonhomologous segment to be "rescued," varies in efficiency with the position of the linearizing cut in the chimeric plasmid DNA, and is a first order reaction with respect to DNA concentration [Contente et al., Plasmid, 2, 555-571 (1979)].

In yeast, restriction enzyme digestion within a region of a circular hybrid plasmid, which region is homologous to chromosomal DNA, greatly enchances the efficiency of integration of plasmid DNA into chromosomal DNA over the efficiency obtainable with circular plasmid DNA [Orr-Weaver et al., Proc. Natl. Acad. Sci. (USA), 78, 6354-6358 (1981)]. By locating the cut site within the region of the plasmid DNA which is homologous to a site on the chromosome, integration of the plasmid may be targeted to the chromosomal site [Orr-Weaver et. al., supra]. The efficiency of transformation is highly dependent on plasmid concentration [Orr-Weaver et. al., supra]. The integration of linear molecules having a deletion within the region of homology is accompanied by repair of the missing information using the homologous region of the chromosome as a template, and the integration process may require the same RAD52 gene product required for double-strand break repair and for gene conversion [Orr-Weaver et al., supra]. However, the insertion of a desired sequence leads to the formation of a direct repeat of the homologous sequence in the chomosome and to the undesirable insertion of vector sequences into the chromosome [Orr-Weaver et. al., Methods Enzymol., 101, 228-245 (1983)]. Moreover, although subsequent recombination of the duplicated sequences may eliminate the undesired duplicate and vector sequences, this elimination occurs at a very low rate [Scherer et al., Proc. Natl. Acad. Sci. (USA), 76, 4951-4955 (1979); and Scherer et. al., Science, 209, 1380-1384 (1980)]. For example, excision of undesired vector sequences was observed in seven out of 900 isolates after 10 generations and in only three of those isolates had wild type gene been deleted to successfully complete replacement by a single copy of an inserted mutant gene [Scherer, Proc. Natl. Acad. Sci. (USA), supra].

The homologous recombination techbique of Orr-Wearer et al., Proc. Natl. Acad. Sci. (USA), supra, may be employed to target gene insertion into mammalian chromosomes, for example to target the insertion of a plasmid containing a beta-globin deletion analog at the beta-globin locus [Smithies et al., Nature, 317, 230-234 (1986). However, as in yeast, the insertion of a desired sequence leads to the formation of a direct repeat of the homologous sequence in the chomosome and to the undesirable insertion of vector sequences therein [Smithies et al., supra]. The low frequency of homologous recombination and the low frequency with which excision events occur limits the potential use of the technique in gene therapy [Maniatis, Nature, 317, 205-206 (1986)].

In B. subtilis, homologous recombination may be employed to insert nonhomologous sequences linked to flanking sequences homologous to the chromosome (and thus to clone a gene if the nonhomologous sequence is a gene) or to delete chromosomal sequences when the flanking sequences are nonadjacent (upon the occurrence of a double crossover recombination event) [Niaudet et al., Gene, 19, 277-284 (1982); and Niaudet et al., J. Bacteriol., 163, 111-120 (1985)]. Introduction of plasmid-borne sequences which are homologous to sequences within a cell but for alterations (i.e., some types or deletions, additions, or inversions) may be cured to complete homology after transformation into the cell [Iglesias et al., Mol. Gen. Genet., 184, 405-409 (1981)]. Nevertheless, the usefulness of these techniques is subject to limitations similar to those present in homologous recombination in yeast.

## Summary of the Invention

The present invention provides a method for insertion or substitution of nucleotides by a process which includes the step of cocultivating within an appropriate host cell both linear target DNA having a break and an oligonucleotide having a nucleotide sequence complementary to a strand of the target DNA on both sides of the break and spanning the break. As employed herein, the term "break" as applied to a segment of DNA, either single- or double-stranded, refers to a separation between ends, including both blunt and cohesive ends. Thus, the term "break" is applied to the separation between ends of single- or double-stranded linear DNA but not to a nick or to a gap in only one strand of double-stranded DNA.

According to the present invention the oligonucleotide includes a first portion (a "portion" being one or more nucleotides in length) completely complementary to the nucleotide sequence of a first region (a "region" being one or more nucleotides in length) of the strand, a second portion completely complementary to the nucleotide sequence of a second region of the strand, and a third portion which is between the first and second portions and which is not complementary to nucleotide sequence of the strand between the first and second regions. Thus, "spanning the break" means bringing the two ends of the target into physical proximity.

The method also includes the step of maintaining said host cell under appropriate conditions, especially conditions favorable for strand break repair, and for a period sufficient to permit incorporation into the target DNA of a third region complementary to the third portion between the first region and the second region. "Incorporation" in the sense in which it is used herein refers to any process by which the desired result is obtained, particularly including filling and ligation and including but not limited to the process of repair.

The cocultivating step of the method for site-directed mutagenesis according to the present invention preferably includes the step of exposing the oligonucleotide to double-stranded target DNA in vitro and more preferably includes the step of cointroducing the oligonucleotide and the target DNA into the host cell. "Introduction" may be any suitable means, including but not limited to injection, CaCl₂ transformation, electroporation, and cell fusion or fusion with liposomes. A cocultivating step further including the step of denaturing the target DNA is presently preferred.

In the site-directed mutagenesis method according to the present invention, DNA on a vector (autonomously replicating DNA or RNA), preferably a plasmid, may be employed as the target. Although a plasmid is preferred, a viral vector such as a bacteriophage may also be employed as convenient.

The exposing step may involve transforming the host cell with the oligonucleotide, transforming the host cell with the target DNA, and, preferably, cotransforming (i.e., transforming at about the same time and especially where the oligonucleotide and the target DNA are included in the same transformation medium) the host cell with the target DNA and with the oligonucleotide. The exposing step may include the step of providing an oligonucleotide having a third portion the most medial nucleotide of which is adjacent a nucleotide of the second portion which is no more than about 26 nucleotides distant from the break along the nucleotide sequence of the strand of the target DNA.

A method for obtaining a deletion of nucleotides according to the present invention includes the step of cocultivating within an appropriate host cell both target DNA having a break, and an oligonucleotide. The sequence of the oligonucleotide is complementary to regions of a strand of the target DNA on both sides of the break and spanning the break, and includes: a first portion completely complementary to the nucleotide sequence of a first region of the strand, a second portion completely complementary to the nucleotide sequence of a second region of the strand and immediately adjacent to the first portion, wherein the target DNA comprises a third region between the first and second regions; and maintaining said host cell under appropriate conditions and for a period sufficient to permit incorporation into the target DNA of a third region complementary to the third portion between the first region and the second region. The method may also have a cocultivating step including the steps of exposing the oligonucleotide to double-stranded target DNA and denaturing the target DNA. The method may have the additional step of providing about a 1000-fold excess of the oligonucleotide as compared to target DNA.

The present invention also provides a method for DNA double-strand break repair. Within an appropriate host cell, target DNA having a double-strand break is cocultivated with an oligonucleotide having a nucleotide sequence complementary to a strand of the target DNA on both sides of the break wherein the oligonucleotide includes a first portion completely complementary to the nucleotide sequence of a first region of the strand and located on a first side of the double-strand break , and a second portion completely complementary to the nucleotide sequence of a second region of the strand and located on a second side of the double-strand break. The host cell is maintained under appropriate conditions and for a period sufficient to permit repair of the double-strand break.

In the method for DNA double-strand break repair according to the present invention, an oligonucleotide may be provided which includes a third portion which is located between the first and second portions and which is not complementary to the strand between the first and the second regions.

The present invention further provides a method for repair of a strand break in single-stranded DNA which involves cocultivating within an appropriate host cell both target DNA having a single-strand break and an oligonucleotide having a nucleotide sequence complementary to a strand of the target DNA on both sides of the break. The oligonucleotide has a first portion completely complementary to the nucleotide sequence of a first region of the strand and located on a first side of the single-strand break , and a second portion completely complementary to the nucleotide sequence of a second region of the strand and located on a second side of the single-strand break. The host cell is maintained in under appropriate conditions and for a period sufficient to permit repair of the single-strand break. The method for DNA single-strand break repair may include a step of providing an oligonucleotide comprising a third portion which is located between the first and second portions and which is not complementary to the strand between the first and the second regions. Thus, the method may be used for site-directed mutagenesis.

According to the present invention, a method for performing gene therapy to obtain a desired genetic modification includes the step of providing cells having a break in target DNA corresponding to the gene to be treated. It also involves the step of introducing into the cells to be treated an oligonucleotide having a nucleotide sequence complementary to a strand of the target DNA on both sides of the break and wherein the oligonucleotide includes a first portion completely complementary to the nucleotide sequence of a second region of the strand, a second portion completely complementary to the nucleotide sequence of a second region of the strand, and a third portion which is between the first and second portions and which is not complementary to nucleotide sequence of the strand between the first and second regions. The host cell is maintained under appropriate conditions and for a period sufficient to permit incorporation of a third region complementary to the third portion between the first region and the second region. The maintaining step may include the step of selecting for cells exhibiting the desired genetic modification.

In order to create a break in the DNA, the cells may be exposed to radiation under conditions suitable for creating a break in DNA by the application of radiation, or by applying a drug to the cells under conditions suitable for creating a break in the DNA by the application of a drug. Most preferably, however, a sequence-specific DNA cleaving molecule having a specificity for the portion of the target DNA which is to be modified is introduced into the cells.

A method for cloning a segment of DNA according to the present invention includes the step of cocultivating within an appropriate host cell vector DNA having a break and linear target DNA to be cloned. Also cocultivated are a first and a second oligonucleotide. The first oligonucleotide includes a first portion completely complementary to the nucleotide sequence of a first region of a strand of the vector DNA, the first region being located on a first side of the break, and a second portion completely complementary to the nucleotide sequence of a first region of a strand of the target DNA, the second region being located proximal to a first end of the target DNA and distal to a second end of the target DNA. The second oligonucleotide has a first portion completely complementary to the nucleotide sequence of a second region of a strand of the vector DNA, the second region being located on a second side of the break, and a second portion completely complementary to the nucleotide sequence of a second region of a strand of the target DNA, said second region being located proximal to the second end of the target DNA and distal to the first end. The host cell is maintained under appropriate conditions and for a period sufficient to permit repair of a break.

Brief Description of the Drawings

Fig. 1 illustrates the sequence of the multicloning site of a vector before and after mutagenesis according to the present invention as well as the homologous sequence of a plasmid resulting from a second mutagenesis according to the present invention;

Fig. 2 is a schematic illustration of the mutagenized vector of Fig. 1 and of the resulting plasmid showing the site of mutagenesis aligned with the mutagenizing oligonucleotide;

Fig. 3 illustrates the sequences of oligonucleotides of various lengths employed according to the present invention;

Fig. 4 is a graphic depiction of the relationship between length of the oligonucleotide employed according to the present invention and both the efficiency of mutagenesis and the number of mutant colonies produced;

Fig. 5 is a graphic depiction of the relationship between the amount of linearized plasmid DNA employed according to the present invention and both the efficiency of mutagenesis and the number of mutant colonies obtained;

Fig. 6 is a graphic depiction of the relationship between the concentration of oligonucleotide used, and both the efficiency of mutagenesis and the number of mutant colonies obtained;

Fig. 7 is a schematic illustration of the sequences of oligonucleotides overlapping the cleavage site and having mutations at various distances from the cleavage site of a target plasmid according to the present invention;

Fig. 8 is a graphic depiction of the relationship between the distance between the cleavage and mutation sites (holding the location of the cleavage site constant), and both the efficiency of mutagenesis and the number of mutant colonies obtained;

Fig. 9 schematically illustrates the sequence of an oligonucleotide aligned with a homologous sequence in a plasmid cleaved at restriction sites as shown; and

Fig. 10 is a graphic depiction of the relationship between the distance between the cleavage and mutation sites (holding the location of the mutation site constant), and both the efficiency of mutagenesis and the number of mutant colonies obtained.

Detailed Description

According to the present invention, an "oligonucleotide", in particular an oligodeoxyribonucleotide having a sequence derived from DNA sequences on the two sides of a DNA double-strand break (i.e. spanning the break) may direct the repair of the break. As a result, the efficiency of transformation of E. coli with denatured linearized plasmid DNA may be increased by orders of magnitude in the presence of the oligonucleotide. If the sequence of the oligonucleotide contains a mutation, the mutation may be incorporated into the repaired plasmid DNA.

The mutagenesis protocol according to the present invention includes a minimal number of steps. Heat-denatured linearized plasmid DNA and the oligonucleotide are introduced by transformation into E. coli, and transformants screened for the mutation. Unlike "bandaid" mutagenesis, the present invention does not require an in vitro, enzymatic reaction (such as ligation), and the present invention is not restricted in its use to DNA having a particular type of end (such as the 3′- and 5′-protruding ends required for "bandaid mutagenesis").

The transformants obtained are screened for the presence of the mutation using methods such as restriction enzyme digestion, colony hybridization, or DNA sequencing.

The procedure for mutagenesis according to the present invention is applicable in situations where plasmid DNA may be linearized in the immediate vicinity of the site to be mutagenized. Where possible, linearization may be accomplished by cleaving DNA at a unique restriction site within a few nucleotides of the residue to be mutated. If a unique restriction site is not available, the following alternative methods of linearization might be used: (i) partial digestion with restriction enzymes which cleave at more than one site; (ii) reconstructing a linear plasmid by the ligation of DNA fragments obtained with other restriction enzymes; or (iii) random cleavage of DNA under mild conditions (e.g. by DNase digestion, or sonication).

The method is particularly well suited to such applications as joining of non-matching ends of plasmid DNA (an alternative to in vitro enzymatic manipulations), destruction and/or introduction of a restriction site, random localized mutagenesis with oligonucleotide probes with degeneracies, or generation of defined deletions following DNA exonuclease digestion. The method has been observed to be successful in rec⁻ host cells but not where the target DNA had no break spanned by the oligonucleotide (e.g. where the target DNA was covalently closed circular DNA).

The method according to the present invention is more specifically described in the following Examples. In Example 1, the method according to the present invention is employed to delete a nucleotide from the pUC9 plasmid. Example 2 illustrates the use of the present invention to insert a nucleotide into the plasmid resulting from the manipulation of Example 1. The relationship between oligonucleotide length and the efficiency of mutagenesis, defined herein as the ratio (percent) of the number of mutants to the total number of transformants obtained, is examined in Example 3. In Example 4, the relationship in the practice of the present invention between the relative amounts of plasmid DNA and oligonucleotide and the efficiency of mutagenesis is explored.

Example 5 illustrates the importance of selecting an oligonucleotide which spans the cleavage site. In Example 6, the efficiency of mutagenesis is determined for variations in the position of the mutation site with respect to a linearization site at a constant position. In Example 7, the relative position of the linearization site is varied with respect to the constant position of a mutation in order to determine the effect on the efficiency of mutagenesis. In Example 8, the relationship between the condition of the linearized DNA (i.e. denatured versus non-denatured) is examined.

In Example 9, the universal applicability of the present invention is illustrated through the use of a variety of plasmids, host strains, restriction enzymes, genes and mutations. Example 10 illustrates the applicability of the present invention to mutagenesis of chromosomal DNA in situ. Example 11 describes the use of the present invention in "gene therapy". In Example 12, the present invention is applied to the cloning of genes.

Example 1

A plasmid system having three properties was sought for a quantitative characterization of the mutagenesis method. The properties were: (i) the presence of a cluster of unique restriction sites for cleaving plasmid DNA; (ii) the plasmid's applicability for color screening of mutant colonies; and (iii) the accuracy of the mutant screening method. The pUC9 plasmid [Vieira et al., Gene 19, 259-268 (1982)] satisfied the first two criteria, because of its multicloning site and because of its indicator gene (a fragment of the lacZ gene encoding the enzyme beta-galactosidase which digests indolyl-beta-D-galactoside present in the medium of cells transformed with the vector to produce a blue pigment). However, the third criterion was not met by pUC9 because both mutations (such as nonsense or frameshift mutations) of the multicloning site within the lacZ gene and cells carrying rearranged plasmids give rise to colonies of the same type (white, on a background of wild-type blue colonies). This made a precise quantitation of mutant colonies difficult.

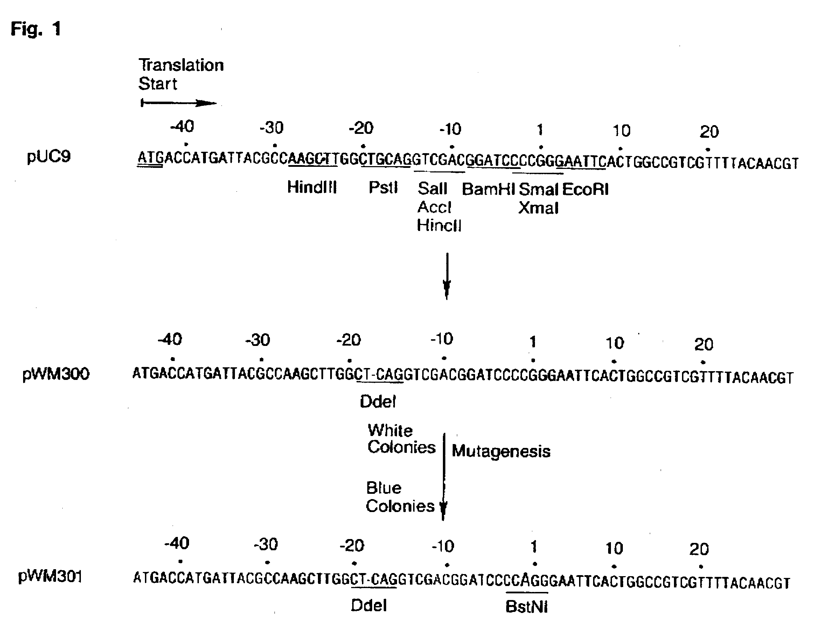

The third requirement was met by constructing a derivative of the pUC9 plasmid carrying a frameshift mutation in the multicloning site. The mutant plasmid, pWM300, was generated using the mutagenesis method according to the present invention, as shown in Fig. 1.

In Fig. 1 sequences of the multicloning site of pUC9 and two derivatized plasmids are shown. Plasmid pWM300, a frameshift mutant of pUC9, was constructed by the DNA double-strand break repair according to the present invention. The oligonucleotide used for mutagenesis encompassed residues -45 to 14 of pWM300 (as numbered in Fig. 1).

Employing the method according to the present invention, pUC9 plasmid DNA was linearized with the PstI restriction enzyme. The completion of the reaction was confirmed by agarose gel electrophoresis. Uncleaved circular plasmid molecules, if used in the procedure, increase the background of the wild-type clones. The plasmid DNA and oligonucleotide were then combined in a denaturation mixture. The denaturation mixture consisted of 50 ng of linearized plasmid DNA and 20 pmol of oligonucleotide in a buffer composed of 10 mM KCl, 5 mM Tris-HCl pH 8.0, 5mM $MgSO_4$, 0.5 mM dithiothreitol in a total volume of 30 $\mu$l in a 1.5 ml Eppendorf tube.

The mixture was incubated at 100°C for 3 min. (in a boiling water bath), the tube was then removed to room temperature for 5 min., the contents of the tube were transferred to 200 $\mu$l of chilled competent cells prepared by $CaCl_2$ treatment [according to Mandel et al., J. Mol. Biol. 53, 159-162 (1970)] and gently mixed. The cells and DNA were incubated on ice for 5 min. and then subjected to a 3 min. heat shock at 37°C. After adding 2 ml of 2 x YT medium [prepared according to Miller, "Experiments in Molecular Genetics", Cold Spring Harbor Laboratory, Cold Spring Harbor, p. 17 (1972)], the cells were incubated at 37°C for 1 hr, then plated on LB plates [prepared according to Miller, "Experiments in Molecular Genetics", Cold Spring Harbor Laboratory, Cold Spring Harbor, NY, p. 433 (1972)] containing 100 $\mu$g/ml ampicillin [GIBCO, Grand Island Biological Company, Grand Island, New York] and 20 $\mu$g/ml of 5-bromo-4-chloro-3-indolyl-$\beta$-D-galactoside (Sigma Chemical Company, St. Louis, Missouri), as appropriate.

Methods for large scale plasmid isolation minipreparations of plasmid DNA and restriction enzyme digestions were performed as described in [Davis et al., "Advanced Bacterial Genetics", Cold Spring Harbor Laborary, Cold Spring Harbor, NY, pp. 116-117 (1980)]. The primer extension method was applied for DNA sequencing, using denatured plasmid DNA as a template according to Chem et al., DNA 4, 165-170 (1985).

## Example 2

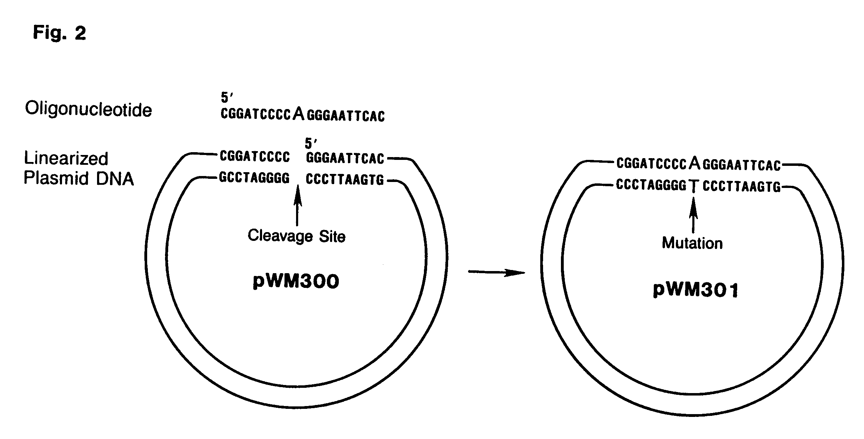

Plasmid pWM300 was employed as a substrate for further mutagenesis. As illustrated in Fig. 2, plasmid pWM300 was simultaneously linearized and blunt-ended by cleavage with the enzyme SmaI. Upon mutagenesis with the oligonucleotide shown in Fig. 2 according to the mutagenesis procedure set forth in Example 1, plasmid pWM301 was created. Thus the coding sequence within the multicloning site was brought in frame to allow for the color screening of transformants. In addition, a BstNI site was generated, which allowed confirmation of the presence of the mutation by restriction analysis. The position of the mutation is indicated by an oversize letter.

As set forth in the following Examples, mutations introduced into the pWM300 system to investigate the mutagenesis method according to the present invention were typically insertions of one residue which restored the reading frame of the lacZ gene fragment. In this system, the mutants formed blue colonies, while cells containing only the parent plasmid (pWM300) and/or rearranged plasmids, gave rise to white colonies.

All insertions introduced into plasmid pWM300 were designed such that they would destroy a unique restriction site within the multicloning site, and at the same time create a new restriction site. This feature allowed for an additional confirmation of the mutagenesis event by restriction digestion of plasmid DNA.

## Example 3

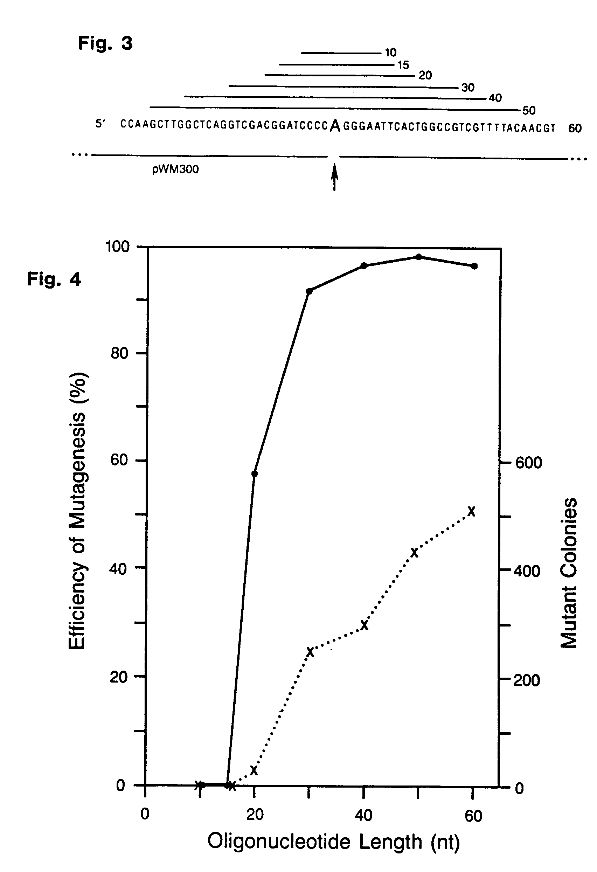

A set of oligonucleotides 10 to 60 residues long was synthesized in order to determine the effect of the length of the oligonucleotide upon the efficiency of mutagenesis. Oligonucleotides were synthesized by the phosphoramidite method [Caruthers, Science 230, 281-285 (1985)] on a model 380A synthesizer available from Applied Biosystems (Foster City, California) and were purified by polyacrylamide gel electrophoresis under denaturing conditions. The sequences of oligonucleotides were homologous to the segment of plasmid pWM300 (as shown in Fig. 1) and were symmetrical with respect to the SmaI site of pWM300, spanning this site. The fragments carried an insertion of one adenine residue into the SmaI site as indicated in Fig. 3. Plasmid pWM300 linearized by digestion with SmaI restriction enzyme was used for mutagenesis.

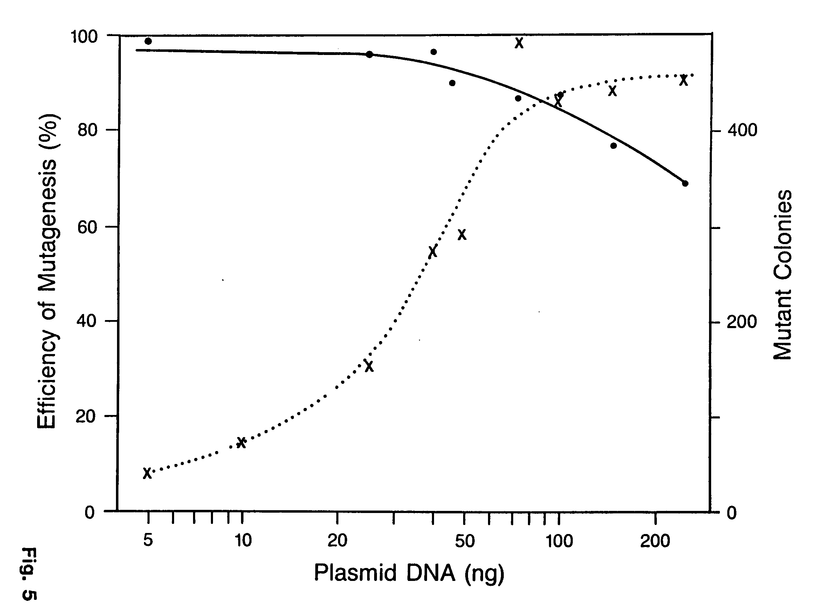

Fig. 3 and Fig. 4 illustrate the role of the length of oligonucleotide in mutagenesis. The sequence of the longest oligonucleotide used (a 60-mer) is given in Fig. 3. Sequences of other oligonucleotides were subsequences of the 60-mer, as indicated by horizontal bars. The lengths of oligonucleotides are given at the right end of each oligomer. The mutation introduced is indicated by the largest letter in the 60-mer. In the solid line representing plasmid pWM300, the SmaI cleavage site is represented by an arrow at a break in the line. Plasmid pWM300 (50 ng) was linearized at the SmaI site and a 20 pmol of each of the oligonucleotides shown in Fig. 3 was used for mutagenesis according to the procedure of Example 1.

In Fig. 4, the results of the experiment are presented. The solid line is a graph of the efficiency of mutagenesis; the dotted line is a graph of the number of mutant colonies per plate. Twenty clones that formed blue colonies were tested by digestions with endonuclease BstNI (BstNI was a newly created restriction site) to confirm the presence of the desired mutation. All 20 clones showed the expected digestion pattern as demonstrated by agarose gel electrophoresis. The DNA sequence of the multicloning site was obtained as described above for two clones. The sequence was as expected for the mutant.

A high efficiency of mutagenesis of over 90% was observed using oligonucleotides of 30 or more residues in length as shown in Fig. 4, and the maximal efficiency was 98% for the 50 residue long oligonucleotide. The 20-residue long oligonucleotide resulted in a half-maximal efficiency, while no mutants were observed using either the 10-mer or the 15-mer. Although the efficiency of mutagenesis seemed to be reaching a plateau using oligonucleotides 40 or more residues in length, the number of mutant colonies continued to increase with the length of DNA. Thus, in order to assure a good efficiency of mutagenesis, an oligonucleotide for use in the method according to the present invention is preferably at least 20 to 30 residues in length, and more preferably longer.

Example 4

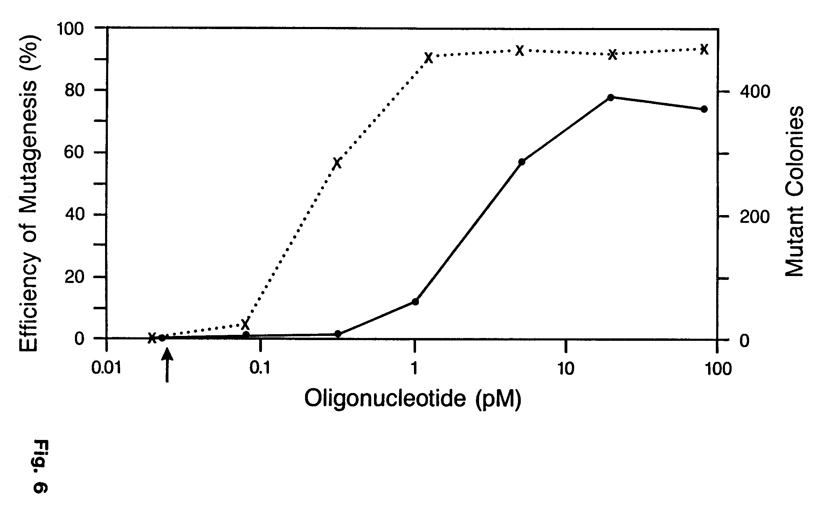

The relationship between the efficiency of mutagenesis according to the procedure of Example 1 and the amount of the linearized plasmid DNA is shown in Fig. 5 in which the solid line illustrates efficiency of mutagenesis; and the dotted line is a graph of the number of mutant colonies per plate.

The pWM300 plasmid linearized at the SmaI site in the amount indicated in Fig. 5 and 20 pmol of the 60-mer depicted in Fig. 3 were used. Titrations with plasmid DNA and oligonucleotide were done to optimize amounts of DNA used for mutagenesis.

As shown in Fig. 5, when the concentration of oligonucleotide was kept constant (20 pmol of the 60-mer per reaction), the efficiency of mutagenesis decreased if over 50 ng of plasmid DNA was used. However, the number of mutants generated increased with the amount of plasmid DNA in the reaction to reach a plateau at about 100 ng DNA. It is preferred, therefore, that about 50 ng of linearized plasmid DNA be used for mutagenesis.

A large molar excess of oligonucleotide over plasmid DNA is important for mutagenesis, as is evident from Fig. 6. In Fig. 6, the indicated amount of the 60-mer of Fig. 3 and 50 ng (approximately 20 fM, as indicated by the arrow) of SmaI-cleaved pWM300 were employed in the procedure according to Example 1. In Fig. 6 the solid line is a graph of the efficiency of mutagenesis and the dotted line is a graph of the number of mutant colonies per plate.

As illustrated in Fig. 6, while the efficiency of mutagenesis reached a plateau at a 1pM concentration of oligonucleotides, the maximal number of mutant colonies was obtained with 20 pmol of oligonucleotide. This amount corresponded to a 1000-fold molar excess of oligonucleotide.

Example 5

In order to better determine the possible mutant-yielding locations of sequence changes within the oligonucleotide and position of the oligonucleotide with respect to the linearization site several experiments were performed employing the mutagenesis procedure of Example 1.

First, in order to determine whether it is important that the oligonucleotide spans the cleavage site, the plasmid pWM300 was linearized with the NdeI restriction enzyme, which cleaves at a site about 240 bp from the multicloning site. It was then tested whether the 60-mer sequence given in Fig. 3 which sequence spans the SmaI site, could induce mutations in the NdeI-cleaved plasmid.

Among 269 transformants no mutants were observed. Typically, if the SmaI-cleaved pWM300 plasmid is used under the same conditions, over 95% of transformants are mutants. The results demonstrate that to obtain mutants it is important that the oligonucleotide spans the linearization site.

Example 6

A set of oligonucleotides was synthesized to define the dependence of mutagenesis according to the procedure of Example 1 on the distance between a mutation and the linearization site.

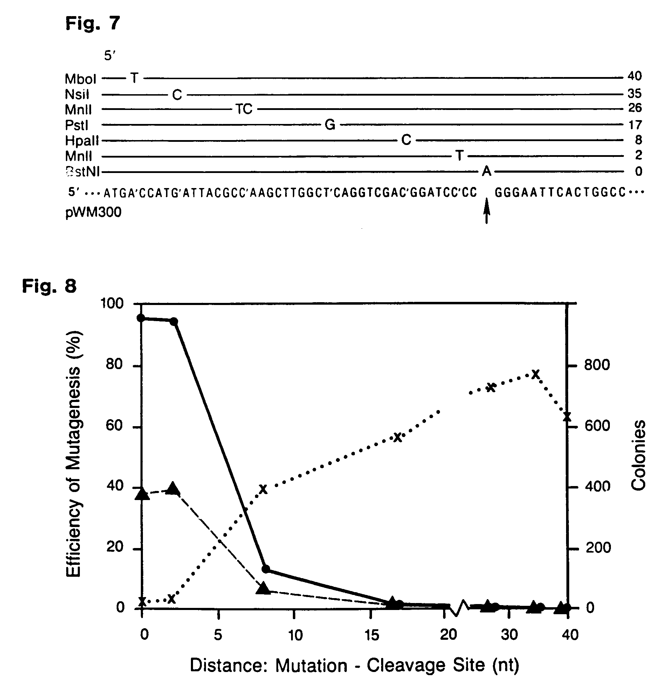

The oligonucleotides were eight symmetrical 60-mers with insertions of one residue at different positions. The sequences of 60-mers which were used for mutagenesis are shown in Fig. 7 as modifications of the sequence of the multicloning site of plasmid pWM300. In Fig. 7, positions of insertion mutations are indicated on horizontal bars representing oligonucleotides. The exact locations of inserted residues are defined by apostrophes on the pWM300 sequence. The insertions generated new restriction sites, which are given at the left end of the line. The oligonucleotide that introduced the MnlI site contained an insertion of one residue combined with a point mutation (...GCCAAG...→...CCTCAG...). The distance between the mutation and the cleavage site is defined as the length of the spacer DNA between the two. The distances (in nucleotides) are given on the right side of the figure.

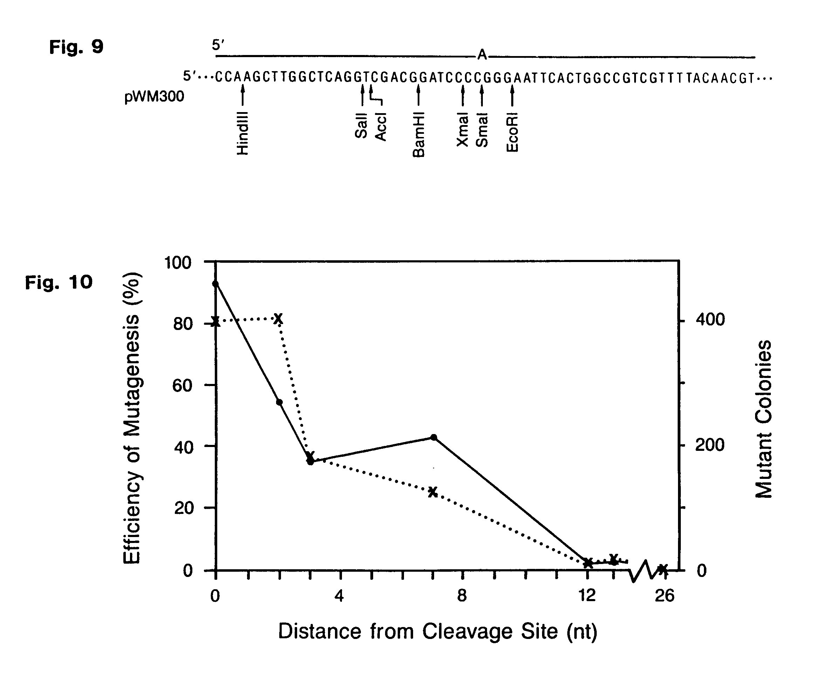

The 60-mers were tested for their ability to generate mutations in the pWM300 plasmid linearized at the SmaI site. The results are illustrated in Fig. 8 in which a solid line graphically depicts the efficiency of mutagenesis, a broken line graphically depicts the number of mutant colonies, and a dotted line graphically depicts the number of non-mutant colonies formed by cells carrying the parent or rearranged plasmids. The results showed that the efficiency of mutagenesis decreased rapidly from 98% to 13% as the distance between the mutation and the cleavage site increased from 0 to 8 base pairs (bp). Half-maximal efficiency was obtained at the distance of about 5 bp.

The efficiency of mutagenesis at the distance of 17 nucleotides (nt) was 1.4% (6 mutants per 422 transformants, confirmed by DNA sequencing). The distance of 26 nt between the mutation and the cleavage site was the maximum that allowed to obtain a mutation, and the efficiency of mutagenesis was 0.2% (2 mutants per 820 transformants, confirmed by DNA sequencing).

In parallel to the decrease in the number of mutants per plate, the number of non-mutant white colonies increased. This strongly suggests that if the mutation is distal to the cleavage site (SmaI), the wild-type sequences around the SmaI recognition sequence on the oligonucleotide can direct the repair and establishment of the plasmid DNA.

Example 7

In an alternative approach to the investigation of Example 6, the position of the linearization site is varied utilizing different restriction enzymes which cleave within the multicloning site.

The 60-mer carrying a mutation at the SmaI site (sequence given in Fig. 3) was used to direct the repair according to the mutagenesis procedure of Example 1. The oligonucleotide used was the 60-mer of Fig. 3, and it is indicated with a heavy line in Fig. 9. Linear plasmid DNA was prepared by digesting pWM300 with one of several restriction endonucleases. Their recognition sites are represented by arrows. One plasmid strand with polarity identical to that of the oligonucleotide of Fig. 1 was arbitrarily chosen to assign locations of the endonuclease cleavage sites.

The results of this experiment, as graphically depicted in Fig. 10, indicate the dependence of the efficiency of mutagenesis (solid line) and number of mutant colonies (dotted line) on the distance between a mutation and the linearization site. The distance is defined here as the length of the spacer DNA between the mutation and the endonuclease cleavage site. As shown in Fig. 10, the mutagenesis efficiency decreased to 40% at the distance of 7 bp and 2% at the distance of 13 bp.

There are several geometric factors to consider when designing an oligonucleotide for mutagenesis. Because a 20-mer was the shortest oligonucleotide for which mutagenesis was observed, the overlap between the oligonucleotide and plasmid DNA on each side of the cleavage site has to be at least 10 nt. Mutants were not obtained if the mutation was located less than 10 nt from the end of oligonucleotide (as indicated in Fig. 8 and in an analogous experiment with 40-mers, data not shown). Evidently, to be incorporated into plasmid DNA the mutation should be at least 10 nt from an end of the oligonucleotide used.

Example 8

9

The relationship between the condition (i.e. denatured versus non-denatured) of the linearized DNA was examined.

When non-denatured, SmaI digested pWM300 plasmid DNA was used for mutagenesis together with the 60-mer (sequence given in Fig. 3) in the procedure of Example 1, only 6 mutants were obtained among a total of 57 transformants. In a parallel experiment, the use of heat-denatured plasmid DNA resulted in 1330 mutants per a total of 1360 transformants.

Thus, for a high efficiency of mutagenesis it is important to denature the linear plasmid DNA before using it for transformation. This is done by incubating a mixture of plasmid DNA and the oligonucleotide at 100°C for 3 min. The use of non-denatured DNA results in a much lower efficiency of mutagenesis.

Example 9

In order to demonstrate that the mutagenesis method according to the present invention is not limited to use with any particular strain, a total of four plasmids, three E. coli strains, three genes and eight restriction enzymes were used in the process of introducing different mutations, as summarized in Table 1. Within the range of ± 10 bp from the cleavage site the mutations were introduced into a plasmid in all attempted cases utilizing the method of mutagenesis described in the Example 1 above.

Four classes of mutations were generated, namely: insertion of one residue, deletion of one residue, point mutation, and point mutation combined with an insertion of one residue. It should be possible to obtain larger insertions, deletions or multiple point mutations utilizing a single oligonucleotide.

**TABLE 1**

**MUTATIONS INTRODUCED**

| Plasmid | Strain | Type of Mutation | Gene/site mutated | Cleavage site | Distance mutation-cleavage site (nt) | Length of oligonucleotide used (nt) | Efficiency (%) |
|---|---|---|---|---|---|---|---|
| pNO1523 | MC1009 | Insertion of 1 residue | rpsL gene | SmaI | 0 | 60 | 88 (7/8) |
|  | CSH26 | " | " | " | " | " | 92 (12/13) |
| pUC9 | JM83 | Deletion of 1 residue | Multicloning site | PstI | 2 | 60 | 25 (1/4) |
| pWM111 | JM83 | Point mutation | C5a gene | PstI | 3 | 59 | 29 (2/7) |
|  | JM83 | " | " | " | 0 | 59 | 57 (4/7) |
|  |  | " | β-lactamase gene | ScaI | 0 | 60 | 25 (4/12) |
| pWM300 | JM83 | Insertion of 1 residue, point mutation, combination of both | Multicloning site | SmaI XmaI EcoRI BamHI SalI, AccI | 0-26 | 20-60 | 0.2-98 |

In Table 1 the number of mutants per a total number of transformants screened by DNA restriction analysis is enclosed within parentheses. The constructions were confirmed by DNA sequencing of the relevant region in at least one clone of each class. All procedures were performed as set forth in Example 1.

For use with pN01523 [Dean, Gene 15, 99-102 (1981)] in host strains MC1009 [Casadaban et al., J. Mol. Biol. 138, 179-207 (1980) or CSH26 [Miller, "Experiments in Molecular Genetics," Cold Spring Harbor Laboratory, Cold Spring Harbor, NY, p. 17 (1972)], the nucleotide sequence of the oligonucleotide was the same as the nucleotide sequence from residue 371 to residue 429 of the non-coding strand of the rpsL gene [Post et al., J. Biol. Chem. 255, 4660-4666 (1980)]. The oligonucleotide contained a mutation that led to the insertion of an A/T base pair between residues 401 and 402, creating a BstNI site.

The construction of the oligonucleotide used for pUC9 [Vieira et al., Gene 19, 259-268 (1982)] in strain JM83 [Vieira et al., Gene 19, 259-268 (1982)] is described in Example 1.

For use with plasmid pWM111 [Mandecki et al., Gene (1986), in press] in strain JM83 where the distance mutation-cleavage site was 3 bases, the oligonucleotide sequence was from residue one to 59 or the non-coding strand of the C5a gene fragment [Mandecki et al., Proc. Natl. Acad. Sci. (USA), 82, 3543-3547 (1985)]. The mutation introduced was an A → G transition at position 24, which created a HaeIII site. For use with plasmid pWM111 where a 59-mer was employed and where the mutation to cleavage site distance was 0, the same oligonucleotide was used, except that the mutation introduced was a C → G transversion at position 27, which created a HpaII site.

For use with plasmid pWM111 where a 60-mer was employed, the oligonucleotide sequence corresponded to residues 3819-3878 of the coding strand of the β-lactamase gene of pBR322 [Sutcliffe, Cold Spring Harbor Symp. Quant. Biol., 43, 77-90 (1978)]. The mutation, a G → A transition at position 3849, destroyed the ScaI and RsaI sites.

The following E. coli strains were employed as host strains in these experiments and in the preceding Examples: CSH26 [Δ(lac pro) ara thi] [Miller, "Experiments in Molecular Genetics," Cold Spring Harbor Laboratory, Cold Spring Harbor, NY, p. 17 (1972)], JM832 [ara, Δ(lac proAB), φ80, lacZΔM15] [Vieira et al., Gene 19, 259-268 (1982) and MC1009 [Δ(lacIPOZY)X74, rpsL, galK, galU, Δ(ara, leu)7697] [Casadaban et al., J. Mol. Biol. 138, 179-207 (1980)]. Plasmids pUC9 [Vieira et al., Gene 19, 259-268 (1982)] and pNO1523 [Dean, Gene 15, 99-102 (1981)] were obtained from Pharmacia, Piscataway, New Jersey, and pWM111 was from the collection of Abbott Laboratories, Abbott Park, Illinois.

Example 10

The procedure of Example 1 may be modified for the purpose of introducing defined mutations into chromosomes in situ.

Cells may be treated in order to induce double-strand breaks through the use of radiation [e.g. according to the procedure of Hutchinson et al., Prog. Nucl. Acid. Res., 32, 115-154 (1985)], or through the use of drugs [e,g, according to the procedure of Walker et al., Ann. Rev. Genet., 19, 103-126 (1985)], or through the use of derivatized DNA probes [e.g., according to the procedures of Dreyer et al., Proc. Natl. Acad. Sci (USA), 82, 968-972 (1985)] and Dervan, Science, 232, 464-471 (1986)]. Oligonucleotides embodying the desired mutation are introduced into the cells, e.g. by CaCl$_2$-mediated transformation as described in Example 1 or electroporation as described in Smithies et al., supra. The cells are maintained under conditions which permit their viability and do not interfere with DNA repair within the cells. The cells are then screened for the presence of the desired mutation [e.g. by Southern blot analysis as set forth in Williams et al., Nature, 310, 476-480 (1984)] after a period of time sufficient to permit DNA repair to occur.

Example 11

Especially in the case where derivatized oligonucleotide probes are employed to target the introduction of double-strand breaks to selected locations in a chromosome, the procedure according to Example 10 may be employed for gene thereapy (i.e. for genetic modification leading to a desired alteration in phenotype.

Specifically, upon identification of a sequence in a portion of a chromosome which may be altered to produce a desired phenotype in a cell or an organism, oligonucleotide probes may be constructed which embody the mutation required to produce the desired phenotype. Cells (including but not limited to egg cells) may be extracted from an organism, or cells of a cell line may be used. A double-strand break may be created in a chromosome within the cells by the use of derivatized DNA probes specific for the site to be mutated according to the procedure of Dreyer et al., supra, or Dervan, supra. More than one strand break may be induced where, for example, a segment of a chromosome is to be deleted or replaced. Oligonucleotides including the desired mutation may then be introduced into the cells by a modification of the procedure of Example 1 or by a number of techniques such as microinjection [see e.g. Brinster et al., Proc. Natl. Acad. Sci. (USA), 82, 4438-4442 (1985)], electroporation [see e.g. Smithies et al., supra], or fusion with a liposome containing the oligonucleotides [see e.g. Lurquin, Nucleic Acids Res., 6, 3773-3784 (1979)]. The cells may then be screened for the presence of the desired mutation and introduced into an organism [see e.g. Williams et al., supra] or cultured as appropriate.

Example 12

The present invention may be applied to DNA clone a DNA segment by covalently joining the DNA segment to a linearized vector by using two oligonucleotides which respectively span a break between a first end of the DNA to be cloned and a first end of a linearized vector, and a sector end of DNA to be cloned and a second end of a linearized vector.

Numerous modifications of and variations in the present invention are expected to occur to those skilled in the art upon consideration of the foregoing specification. For example, although the term oligonucleotide is employed herein, it will be obvious to one skilled in the art to employ any linear, single-stranded deoxyribonucleic acid in the methods according to the present invention. Likewise, it will be readily apparent to one skilled in the art that the present invention may be employed to insert, delete and substitute (i.e., simultaneously delete and insert) groups of nucleotides constituting one or more codons in order to obtain genes encoding analogs of polypeptides from a gene encoding a naturally occurring form.

Consequently, it is intended that the invention be limited only to the extent reflected by the appended claims.

**Claims**

1. A method for insertion or substitution of nucleotides by a process comprising the steps of:
cocultivating within an appropriate host cell both target DNA having a break, and an oligonucleotide having a nucleotide sequence complementary to a strand of the target DNA on both sides of the break and spanning the break, wherein the oligonucleotide comprises:
a first portion completely complementary to the nucleotide sequence of a first region of the strand,
a second portion completely complementary to the nucleotide sequence of a second region of the strand, and
a third portion which is between the first and second portions and which is not complementary to nucleotide sequence of the strand between the first and second regions; and
maintaining said host cell under appropriate conditions and for a period sufficient to permit incorporation into the target DNA of a third region complementary to the third portion between the first region and the second region.

2. The method as recited in claim 1 wherein said cocultivating step comprises the step of cointroducing the oligonucleotide and the target DNA into the host cell.

3. The method as recited in claim 2 wherein said cointroduction step comprises the step of cotransforming the host cell with the target DNA and with the oligonucleotide.

4. The method as recited in claim 3 wherein said cocultivating step comprises the steps of exposing the oligonucleotide to double-stranded target DNA and denaturing the target DNA.

5. The method as recited in claim 1 further comprising the step of employing DNA on a vector as the target DNA.

6. The method as recited in claim 1 further comprising the step of providing about a 1000-fold excess of the oligonucleotide as compared to target DNA.

7. The method as recited in claim 1 wherein said cocultivating step comprises the step of providing an oligonucleotide having a third portion the most medial nucleotide of which is adjacent to a nucleotide of the second portion which is complementary to a nucleotid in the target DNA that is no more than about 26 nucleotides distant from the site of the break in the nucleotide sequence of the target DNA.

8. The method as recited in claim 1 wherein said cocultivating step comprises the step of furnishing an oligonucleotide in which the first and second portions are each about 10 nucleotides or more in length.

9. A method for obtaining a deletion by site-directed mutagenesis comprising the steps of:
cocultivating within an appropriate host cell both target DNA having a break, and an oligonucleotide having a nucleotide sequence complementary to regions of a strand of the target DNA on both sides of the break and spanning the break, and wherein the oligonucleotide comprises:

13

EP 0 253 193 B1

a first portion completely complementary to the nucleotide sequence of a first region of the strand,

a second portion completely complementary to the nucleotide sequence of a second region of the strand and immediately adjacent to the first portion, wherein the target DNA comprises a third region between the first and second regions; and

maintaining said host cell under appropriate conditions and for a period sufficient to permit incorporation into the target DNA of a third region complementary to the third portion between the first region and the second region.

10. The method as recited in claim 9 wherein said cocultivating step comprises the steps of exposing the oligonucleotide to double-stranded target DNA and denaturing the target DNA.

11. The method as recited in claim 9 further comprising the step of providing about a 1000-fold excess of the oligonucleotide as compared to target DNA.

12. A method for DNA double-strand break repair comprising the steps of:

cocultivating within an appropriate host cell both target DNA having a double-strand break and an oligonucleotide having a nucleotide sequence complementary to a strand of the target DNA on both sides of the break wherein the oligonucleotide comprises:

a first portion completely complementary to the nucleotide sequence of a first region of the strand and located on a first side of the double-strand break , and

a second portion completely complementary to the nucleotide sequence of a second region of the strand and located on a second side of the double-strand break; and

maintaining the host cell under appropriate conditions and for a period sufficient to permit repair of the double-strand break.

13. The method for DNA double-strand break repair as recited in claim 12 further comprising the step of providing an oligonucleotide comprising a third portion which is located between the first and second portions and which is not complementary to the strand between the first and the second regions.

14. A method for repair of a strand break in single-stranded DNA comprising the steps of:

cocultivating within an appropriate host cell both single-stranded target DNA having a single-strand break and an oligonucleotide having a nucleotide sequence complementary to a strand of the target DNA on both sides of the break wherein the oligonucleotide comprises:

a first portion completely complementary to the nucleotide sequence of a first region of the strand and located on a first side of the single-strand break , and

a second portion completely complementary to the nucleotide sequence of a second region of the strand and located on a second side of the single-strand break; and

maintaining the host cell under appropriate conditions and for a period sufficient to permit repair of the single-strand break.

15. The method for DNA single-strand break repair as recited in claim 14 further comprising the step of providing an oligonucleotide comprising a third portion which is located between the first and second portions and which is not complementary to the strand between the first and the second regions.

16. A method for performing gene treatment to obtain a genetic modification and a consequent phenotypic modification comprising the steps of:

providing cells having a break in target DNA corresponding to the gene encoding the undesirable phenotype to be modified

introducing into the cells to be treated an oligonucleotide having a nucleotide sequence complementary to a strand of the target DNA on both sides of the break and wherein the oligonucleotide comprises:

a first portion completely complementary to the nucleotide sequence of a first region of the strand,

a second portion completely complementary to the nucleotid sequence of a second region of the strand, and

a third portion which is between the first and second portions and which is not complementary to nucleotide sequence of the strand between the first and second regions; and

maintaining said host cell under appropriate conditions and for a period sufficient to permit incorporation of a third region complementary to the third portion between the first region and the second region so that the phenotype is modified.

14

**17.** The method as recited in claim 16 wherein said providing step comprises exposing the cells to radiation under conditions suitable for creating a break in DNA by the application of radiation.

**18.** The method as recited in claim 16 wherein said providing step comprises the step of applying a drug to the cells under conditions suitable for creating a break in the DNA by the application of the drug.

**19.** The method as recited in claim 17 wherein said providing step comprises introducing into the cells a sequence-specific DNA cleaving molecule having a specificity for the portion of the target DNA which is to be modified.

**20.** The method as recited in claim 16 wherein said maintaining step comprises the step of selecting for cells exhibiting the desired genetic modification.

**21.** A method for cloning a segment of DNA comprising the steps of:
cocultivating within an appropriate host cell vector DNA having a break, linear DNA to be cloned, a first oligonucleotide comprising:
a first portion completely complementary to the nucleotide sequence of a first region of a strand of the vector DNA, said first region being located on a first side of the break, and
a second portion completely complementary to the nucleotide sequence of a first region of a strand of the linear DNA to be cloned, said second region being located proximal to a first end of the linear DNA to be cloned and distal to a second end of the linear DNA to be cloned; and
a second oligonucleotide comprising:
a first portion completely complementary to the nucleotide sequence of a second region of a strand of the vector DNA, said second region being located on a second side of the break, and
a second portion completely complementary to the nucleotide sequence of a second region of a strand of the linear DNA to be cloned, said second region being located proximal to the second end of the linear DNA to be cloned and distal to the first end; and
maintaining the host cell under appropriate conditions and for a period sufficient to permit repair of a break.

**22.** A method for site-directed mutagenesis comprising the steps of:
providing target DNA on a plasmid linearized at a break, and an oligonucleotide having a nucleotide sequence complementary to a strand of the target DNA on both sides of the break and spanning the break, and wherein the oligonucleotide comprises:
a first portion completely complementary to the nucleotide sequence of a first region of the strand,
a second portion completely complementary to the nucleotide sequence of a second region of the strand, and
a third portion which is between the first and second portions and which is not complementary to nucleotide sequence of the strand between the first and second regions the most medial nucleotide of the third portion being adjacent to a nucleotide of the second portion which is complementary to a nucleotide in the strand that is less than about 26 nucleotides distant from the break along the nucleotide sequence of the strand;
exposing the oligonucleotide to denatured double-stranded target DNA;
cotransforming an appropriate microbial host cell with the target DNA and with the oligonucleotide;
cocultivating within the appropriate microbial host cell both the target DNA and the oligonucleotide; and
maintaining said host cell under appropriate conditions and for a period sufficient to permit incorporation into the target DNA of a third region complementary to the third portion between the first region and the second region.

**Patentansprüche**

**1.** Verfahren zur Insertion oder Substitution von Nukleotiden durch einen Prozess, umfassend den Schritten:
Cokultivierung in einer geeigneten Wirtszelle sowohl der Ziel-DNA mit einer Bruchstelle als auch eines Oligonukleotids mit einer auf beiden Seiten der Bruchstelle zu einem Strang der Ziel-DNA komplementären Nukleotidsequenz und das die Bruchstelle überspannt, wobei das Oligonukleotid
einen zur Nukleotidsequenz einer ersten Strangregion völlig komplementären ersten Teil,

einen zur Nukleotidsequenz einer zweiten Strangregion völlig komplementären zweiten Teil und einen dritten Teil, der zwischen dem ersten und dem zweiten Teil liegt und der nicht komplementär zur Nukleotidsequenz des Strangs zwischen der ersten und der zweiten Region ist, umfaßt; und

Halten der genannten Wirtszelle unter geeigneten Bedingungen und fur einen Zeitraum, der ausreicht, um die Inkorporation einer zum dritten Teil zwischen der ersten Region und der zweiten Region komplementären dritten Region in die Ziel-DNA zu ermöglichen, umfaßt.

2. Verfahren nach Anspruch 1, wobei der genannte Cokultivierungsschritt den Schritt der Cointroduktion des Oligonukleotids und der Ziel-DNA in die Wirtszelle umfaßt.

3. Verfahren nach Anspruch 2, wobei der genannte Schritt der Cointroduktion den Schritt der Cotransformation der Wirtszelle mit der Ziel-DNA und dem Oligonukleotid umfaßt.

4. Verfahren nach Anspruch 3, wobei der genannte Cokultivierungsschritt die Schritte der Exposition des Oligonukleotids gegenüber der doppelsträngigen Ziel-DNA und der Denaturierung der Ziel-DNA umfaßt.

5. Verfahren nach Anspruch 1, das außerdem den Schritt des Einsatzes von DNA auf einem Vektor als die Ziel DNA umfaßt.

6. Verfahren nach Anspruch 1, das außerdem den Schritt der Bereitstellung eines im Vergleich zur Ziel-DNA etwa 1000fachen Überschußes des Oligonukleotides umfaßt.

7. Verfahren nach Anspruch 1, wobei der genannte Cokultivierungsschritt den Schritt der Bereitstellung eines Oligonucleotids mit einem dritten Teil, bei dem sich das medialste Nucleotid unmittelbar an ein Nucleotid des zweiten Teils anschließt, das zu einem nicht mehr als 26 Nukleotide von der Bruchstelle in der Nukleotidsequenz der Ziel-DNA entfernten Nucleotid komplementär ist, umfaßt.

8. Verfahren nach Anspruch 1, wobei der genannte Cokultivierungsschritt den Schritt der Erzeugung eines Oligonucleotids, in dem die Länge des ersten und zweiten Teils jeweils etwa 10 Nucleotide oder mehr beträgt, umfaßt.

9. Verfahren, bei dem eine Deletion durch ortsgerichtete (site-directed) Mutagenese erhalten wird, umfassend den Schritten:
Cokultivierung in einer geeigneten Wirtszelle sowohl von Ziel-DNA mit einer Bruchstelle als auch eines Oligonukleotids mit einer auf beiden Seiten der Bruchstelle zu einem Strang der Ziel-DNA komplementären Nukleotidsequenz, das die Bruchstelle überspannt, wobei das Oligonukleotid
einen zur Nukleotidsequenz einer ersten Strangregion völlig komplementären ersten Teil, einen zur Nukleotidsequenz einer zweiten Strangregion völlig komplementären und unmittelbar an den ersten Teil anschließenden zweiten Teil, worin die Ziel-DNA eine dritte Region zwischen der ersten und der zweiten Region unfaßt, umfaßt; und
Halten der genannten Wirtszelle unter geeigneten Bedingungen und für einen Zeitraum der ausreicht, um die Inkorporation einer zum dritten Teil, zwischen der ersten Region und der zweiten Region, komplementären dritten Region in die Ziel-DNA zu emöglichen.

10. Verfahren nach Anspruch 9, wobei der genannte Cokultivierungsschritt die Schritte der Exposition des Oligonukleotids gegenüber der doppelsträngigen Ziel-DNA und der Denaturierung der Ziel-DNA umfaßt.

11. Verfahren nach Anspruch 9, das weiterhin den Schritt der Bereitstellung eines im Vergleich zur Ziel-DNA etwa 1000fachen Überschußes des Oligonukleotides umfaßt.

12. Verfahren zur DNA-Doppelstrang-Bruchstellenreparatur, umfassend den Schritten
Cokultivierung in einer geeigneten Wirtszelle sowohl von Ziel-DNA mit einer Doppelstrang-Bruchstelle als auch eines Oligonukleotids mit einer auf beiden Seiten der Bruchstelle zu einem Strang der Ziel-DNA komplementären Nukleotidsequenz, wobei das Oligonukleotid
einen zur Nukleotidsequenz einer ersten Strangregion völlig komplementären und auf einer ersten Seite der Doppelstrang-Bruchstelle gelegenen ersten Teil und
einen zur Nukleotidsequenz einer zweiten Strangregion völlig komplementären und auf einer

16

zweiten Seite der Doppelstrang-Bruchstelle gelegenen zweiten Teil, umfaßt; und
Halten der Wirtszelle unter geeigneten Bedingungen und für einen Zeitraum, der eine Reparatur der Doppelstrang-Bruchstelle zuläßt, umfaßt.

13. Verfahren zur DNA-Doppelstrang Bruchstellenreparatur nach Anspruch 12, das weiterhin den Schritt der Bereitstellung eines Oligonukleotids umfaßt, das einen dritten Teil, der zwischen dem ersten und dem zweiten Teil liegt und der nicht komplementär zum Strang zwischen der ersten und der zweiten Region ist, umfaßt.

14. Verfahren zur Reparatur eines Strangbruches in Einzelstrang-DNA, umfassend den Schritten:
Cokultivierung in einer geeigneten Wirtszelle sowohl von Einzelstrang-Ziel-DNA mit einer Einzelstrang-Bruchstelle als auch von einem Oligonukleotid mit einer auf beiden Seiten der Bruchstelle zu einem Strang der Ziel-DNA komplementären Nukleotidsequenz, wobei das Oligonukleotid
einen zur Nukleotidsequenz einer ersten Strangregion völlig komplementären ersten Teil, gelegen auf einer ersten Seite des Einzelstrang-Bruches und
einen zur Nukleotidsequenz einer zweiten Strangregion völlig komplementären zweiten Teil, gelegen auf einer zweiten Seite des Einzelstrang-Bruches umfaßt und
Halten der genannten Wirtszelle unter geeigneten Bedingungen und für einen Zeitraum, der ausreicht, um eine Reparatur des Einzelstrangbruches zu ermöglichen.

15. Verfahren zur DNA-Einzelstrang Bruchreparatur nach Anspruch 14, das weiterhin den Schritt der Bereitstellung eines Oligonukleotids umfaßt, das einen dritten Teil, der zwischen dem ersten und dem zweiten Teil liegt und der nicht komplementär zum Strang zwischen der ersten und der zweiten Region ist, umfaßt.

16. Verfahren zur Durchführung einer Gen-Behandlungzum Erhalt einer genotypischen Modifikation und einer anschließenden phänotypischen Modifikation, umfassend den Schritten:
Einführung eines Oligonukleotids mit einer zu einem Strang der Ziel-DNA auf beiden Seiten des Bruches komplementären Nukleotidsequenz in die zu behandelnden Zellen, und wobei das Oligonukleotid
einen zur Nukleotidsequenz einer ersten Strangregion völlig komplementären ersten Teil,
einen zur Nukleotidsequenz einer zweiten Strangregion völlig komplementären zweiten Teil und
einen dritten Teil, der zwischen dem ersten und dem zweiten Teil liegt und der nicht komplementär zur Nukleotidsequenz des Strangs zwischen der ersten und der zweiten Region ist, umfaßt; und
Halten der genannten Wirtszelle unter geeigneten Bedingungen und für einen Zeitraum der ausreicht, um eine Inkorporation einer zum dritten Teil zwischen der ersten und der zweiten Region komplementären dritten Region zu ermöglichen, so daß der Phänotyp modifiziert wird.

17. Verfahren nach Anspruch 16, wobei der genannte Bereitstellungsschritt die Exposition der Zellen gegenüber einer Strahlung unter Bedingungen, die zur Erzeugung einer Bruchstelle in DNA durch Anwendung der Strahlung geeignet sind, umfaßt.

18. Verfahren nach Anspruch 16, wobei der genannte Bereitstellungsschritt den Schritt der Anwendung einer Droge auf die Zellen, unter Bedingungen, die zur Erzeugung einer Bruchstelle in der DNA durch die Anwendung der Droge geeignet sind, umfaßt.

19. Verfahren nach Anspruch 17, wobei der genannte Bereitstellungsschritt die Einführung eines sequenzspezifischen DNA-Spaltungsmoleküls mit einer spezifischen Affinität gegenüber dem zu modifizierenden Teil der Ziel-DNA umfaßt.

20. Verfahren nach Anspruch 16, wobei der genannte Schritt der Zellhaltung den Schritt der Selektion von Zellen, die die gewünschte genetische Modifikation aufweisen, umfaßt.

21. Verfahren zur Klonierung eines DNA-Segments, umfassend den Schritten:
Cokultivierung eines zur zu klonierenden DNA linearen ersten Oligonukleotids in einer geeigneten Wirtszellen-Vektor-DNA mit einer Bruchstelle, das
einen zur Nukleotidsequenz einer ersten Strangregion der Vektor-DNA völlig komplementären ersten Teil, wobei die genannte erste Region auf einer ersten Seite des Bruchs liegt und

einen zur Nukleotidsequenz einer ersten Strangregion der linearen, zu klonierenden DNA völlig komplementären zweiten Teil, wobei die genannte zweite Region proximal zu einem ersten Ende der zu klonierenden linearen DNA und distal zu einem zweiten Ende der zu klonierenden linearen DNA liegt,umfaßt; und

ein zweites Oligonukleotid, das

einen zur Nukleotidsequenz einer zweiten Strangregion der Vektor-DNA völligkomplementären ersten Teil, wobei sich die genannte zweite Region auf einer zweiten Seite der Bruchstelle befindet, und

einen zur Nukleotidsequenz eines zweiten Stranges der zu klonierenden linearen DNA völlig komplementären zweiten Teil, wobei die genannte zweite Region proximal zum zweiten Ende der zu klonierenden linearen DNA und distal zum ersten Ende liegt, umfaßt; und

Halten der Wirtszelle unter geeigneten Bedingungen und für einen Zeitraum, der ausreicht, um eine Bruchstellenreparatur zu ermöglichen.

**22.** Verfahren zur ortsspezifischen (site-directed) Mutagenese, umfassend den Schritten:

Bereitstellung von Ziel-DNA auf einem an einer Bruchstelle linearisierten Plasmid und eines Oligonukleotids mit einer zu einem Strang der Ziel-DNA auf beiden Seiten der Bruchstelle komplementären Nukleotidsequenz und das die Bruchstelle überspannt und wobei das Oligonukleotid

einen zur Nukleotidsequenz einer ersten Strangregion völlig komplementären ersten Teil,

einen zur Nukleotidsequenz einer zweiten Strangregion völlig komplementären zweiten Teil und

einen dritten Teil, der zwischen dem ersten und dem zweiten Teil liegt und der nicht zur Nukleotidsequenz des Stranges zwischen der ersten und der zweiten Region komplementär ist, wobei das medialste Nukleotid des dritten Teils sich unmittelbar an ein Nukleotid des zweiten Teils anschließt, das komplementär zu einem Nukleotid in dem Strang ist, das weniger als etwa 26 Nukleotide von der Bruchstelle entlang der Nukleotidsequenz des Strangs entfernt ist, umfaßt;

Exposition des Oligonukleotids gegenüber denaturierter, doppelsträngiger Ziel-DNA;

Cotransformation einer passenden Mikroorganismus-Wirtszelle mit der Ziel-DNA und mit dem Oligonukleotid;

der Cokultivierung sowohl der Ziel-DNA und des Oligonukleotids in der passenden Mikroorganismus-Wirtszelle;

und

Halten der genannten Wirtszelle unter geeigneten Bedingungen und für einen Zeitraum, der ausreicht, um Inkorporation einer zum dritten Teil zwischen der ersten und der zweiten Region komplementären dritten Region zu ermöglichen.

## Revendications

**1.** Une méthode pour l'insertion ou la substitution de nucléotides par un procédé comprenant les étapes de:

co-culture dans une cellule hôte appropriée de, à la fois, l'ADN cible présentant une cassure, et un oligonucléotide ayant une séquence de nucléotide complémentaire d'un brin de l'ADN cible sur les deux bord de la cassure et enjambant la cassure, dans laquelle l'oligonucléotide comprend:

une première portion complètement complémentaire de la séquence de nucléotide d'une première région du brin,

une seconde portion complètement complémentaire de la séquence de nucléotide d'une seconde région du brin, et

une troisième portion qui est entre les première et seconde portion et qui n'est pas complémentaire de la séquence de nucléotide du brin entre les première et seconde régions; et

maintien de ladite cellule hôte dans des conditions appropriées et pendant une période suffisante pour permettre l'incorporation dans l'ADN cible d'une troisième région complémentaire de la troisième portion entre la première te la seconde région.

**2.** La méthode telle qu'elle est exposée dans la revendication 1 dans laquelle ladite étape de co-culture comprend l'étape de co-introduction de l'oligonucléotide et de l'ADN cible dans la cellule hôte.

**3.** La méthode telle qu'elle est exposée dans la revendication 2 dans laquelle ladite étape de co-introduction comprend l'étape de co-transformation la cellule hôte avec l'ADN cible et avec l'oligonucléotide.

4. La méthode comme elle est exposée dans la revendication 3 dans laquelle l'étape de co-culture comprend les étapes d'exposition de l'oligonucléotide à l'ADN cible double brin et de dénaturation de l'AN cible.

5. La méthode comme elle est exposée dans la revendication 1 comprenant en outre l'étape de l'utilisation de l'ADN sur un vecteur comme l'ADN cible.

6. La méthode comme elle est exposée dans la revendication 1 comprenant en outre l'étape de founir un excès d'environ 1000 fois de l'oligonucléotide par rapport à l'ADN cible.

7. La méthode comme elle est exposée dans la revendication 1 dans laquelle ladite étape de co-culture comprend l'étape de fournir un oligonucléotide ayant une troisième portion dont le nucléotide le plus médian est adjacent à un nucléotide de la seconde portion qui est complémentaire d'un nucléotide dans l'ADN cible qui est lui-même situé à une distance égale ou inférieure d'environ 26 nucléotides à partir du site de la cassure dans la séquence de nucléotides de l'ADN cible.

8. La méthode comme elle est exposée dans la revendication 1 dans laquelle ladite étape de co-culture comprend l'étape de fournir un oligonucléotide dans lequel les première et seconde portions sont longues chacune d'environ 10 nucléotides ou plus.

9. Une méthode pour obtenir une délétion par mutagénèse dirigée sur un site comprenant les étapes de:
co-culture dans une cellule hôte appropriée à la fois d'ADN cible présentant une cassure, et d'un oligonucléotide ayant une séquence de nucléotides complémentaire de régions d'un brin de l'ADN cible sur les deux bords de la cassure et enjambant la cassure, et dans laquelle l'oligonucléotide comprend:
une première portion complètement complémentaire de la séquence de nucléotides d'une première région du brin,
une seconde portion complètement complémentaire de la séquence de nucléotides d'une seconde région du brin et immédiatement adjacente à la première portion, dans laquelle l'ADN cible comprend une troisième région entre les première et seconde régions; et
maintient de ladite cellule hôte dans des conditions appropriées et pendant une période suffisante pour permettre l'incorporation dans l'ADN cible d'une troisième région complémentaire de la troisième portion entre la première région et la seconde région.

10. La méthode comme elle est exposée dans la revendication 9 dans laquelle ladite étape de co-culture comprend les étapes d'exposition de l'oligonucléotide à l'ADN cible double brin et de dénaturation de l'ADN cible.

11. La méthode comme elle est exposée dans la revendication 9 comprenant en outre l'étape de fournir un excès d'environ 1000 fois d'oligonucléotide par rapport à l'ADN cible.

12. Une méthode pour réparer une cassure sur le double brin de l'ADN comprenant les étapes de:
co-culture dans une cellule hôte appropriée de, à la fois, l'ADN cible présentant une cassure sur les deux brins et un oligonucléotide ayant une séquence de nucléotides complémentaires d'un brin de l'ADN cible sur les deux bords de la cassure dans laquelle l'oligonucléotide comprend:
une première portion complètement complémentaire de la séquence de nucléotides d'une première région du brin et localisée sur un premier bord de la cassure des deux brins, et
une seconde portion complètement complémentaire de la séquence de nucléotides d'une seconde région du brin et localisée sur un second bord de la cassure des deux brins; et
maintien de la cellule hôte dans les conditions appropriées et pour une période suffisante pour permettre la réparation de la cassure sur les deux brins.

13. La méthode pour réparer une cassure sur les deux brins d'un ADN comme il est exposé dans la revendication 12 comprenant en outre l'étape de fournir un oligonucléotide comprenant une troisième portion qui est localisée entre les première et seconde portions et qui n'est pas complémentaire du brin entre les première et seconde régions.

**14.** Une méthode pour la réparation d'une cassure d'un brin dans un ADN simple brin comprenant les étapes de:

co-culture dans une cellule hôte appropriée de, à la fois un ADN cible à simple brin ayant une cassure sur le brin unique et un oligonucléotide ayant une séquence de nucléotides complémentaire à un brin de l'ADN cible sur les deux bords de la cassure dans laquelle l'oligonucléotide comprend:

une première portion complètement complémentaire d'une séquence de nucléotides d'une première région du brin et localisée sur un premier bord de la cassure du brin unique, et

une seconde portion complètement complémentaire de la séquence de nucléotides d'une seconde région du brin et localisée sur un second bord de la cassure du brin unique; et

maintien de la cellule hôte dans des conditions appropriées et pour une période suffisante pour permettre la réparation de la cassure sur le brin unique.

**15.** La méthode pour réparer une cassure d'un ADN simple brin comme il est exposé dans la revendication 14 comprenant en outre l'étape de fournir un oligonucléotide comprenant une troisième portion qui est localisée entre les première et seconde portions et qui n'est pas complémentaire du brin entre les première et seconde régions.

**16.** Une méthode pour réaliser un traitement d'un gène pour obtenir une modification génétique et en conséquence une modification phénotypique comprenant les étapes de:

fournir des cellules présentant une cassure dans l'ADN cible correspondant au gène codant le phénotype indésirable à modifier

introduire dans les cellules à traiter un oligonucléotide ayant une séquence de nucléotides complémentaire au brin de l'ADN cible sur les deux bords de la cassure et dans lesquelles l'oligonucléotide comprend:

une première portion complètement complémentaire de la séquence de nucléotides d'une première région du brin,

une seconde portion complètement complémentaire de la séquence de nucléotides d'une seconde région du brin, et

une troisième portion qui est entre les première et seconde portions et qui n'est pas complémentaire de la séquence de nucléotides du brin entre les première et seconde régions; et

maintenir ladite cellule hôte dans des conditions appropriées et pendant une période suffisante pour permettre l'incorporation d'une troisième région complémentaire de la troisième portion entre la première région et la seconde région de sorte que le phénotype soit modifié.

**17.** La méthode comme elle est exposée dans la revendication 16 dans laquelle ladite étape qui consiste à fournir des cellules comprend d'exposer les cellules à une radiation dans des conditions convenables pour provoquer une cassure dans l'ADN par l'application d'une radiation.

**18.** La méthode come elle est exposée dans la revendication 16 dans laquelle ladite étape de fournir des cellules comprend l'étape d'appliquer un agent chimique aux cellules dans des conditions convenables pour provoquer une cassure dans l'ADN du fait de l'application de l'agent chimique.

**19.** La méthode comme elle est exposée dans la revendication 17 dans laquelle ladite étape de fournir des cellules comprend l'introduction dans les cellules d'une molécule coupant une séquence spécifique d'ADN ayant une spécificité pour la portion de l'ADN cible qui doit être modifiée.

**20.** La méthode comme elle est exposée dans la revendication 16 dans laquelle l'étape de maintien comprend l'étape de sélection des cellules qui présentent la modification génétique désirée.

**21.** Une méthode pour cloner un segment d'ADN comprenant les étapes de:

co-culture dans une cellule hôte appropriée d'un ADN vecteur ayant une cassure, un ADN linéaire à cloner, un premier oligonucléotide comprenant:

une première portion complètement complémentaire de la séquence de nucléotides d'une première région d'un brin de l'ADN vecteur, ladite première région étant localisée sur un premier bord de la cassure, et

une seconde portion complètement complémentaire de la séquence de nucléotides d'une première région d'un brin de l'ADN linéaire à cloner, ladite seconde région étant localisée à proximité d'une première extrémité d'un ADN linéaire à cloner et distante de la deuxième extrémité de l'ADN linéaire à cloner; et

un second oligonucléotide comprenant:

une première portion complètement complémentaire de la séquence de nucléotides d'une seconde région du brin de l'ADN vecteur, ladite seconde région étant située sur un second bord de la cassure, et

une seconde portion complètement complémentaire de la séquence de nucléotides d'une seconde région d'un brin de l'ADN linéaire à cloner, ladite seconde région étant située à proximité de la seconde extrémité de l'ADN linéaire à cloner et à distance de la première extrémité; et

maintien de la cellule hôte dans des conditions appropriées et pendant une période suffisante pour permettre la réparation de la cassure.

22. Une méthode pour une mutagénèse dirigée sur un site comprenant les étapes de:

fournir un ADN cible sur un plasmide linéarisé à une cassure, et un oligonucléotide ayant une séquence de nucléotides complémentaire à un brin de l'ADN cible sur les deux bords de la cassure et enjambant la cassure, et dans lequel l'oligonucléotide comprend:

une première portion complètement complémentaire de la séquence de nucléotides d'une première région du brin,

une seconde portion complètement complémentaire de la séquence de nucléotides d'une seconde région du brin, et

une troisième portion qui est située entre les première et seconde portions et qui n'est pas complémentaire de la séquence de nucléotides du brin entre les première et seconde régions

le nucléotide le plus médian de la troisième portion étant adjacent à un nucléotide de la seconde portion qui est complémentaire d'un nucléotide dans le brin qui est à une distance inférieure ou égale d'environ 26 nucléotides de la cassure le long de la séquence de nucléotides du brin;

exposition de l'oligonucléotide à l'ADN cible double brin dénaturé;

co-transformation d'une cellule hôte microbienne appropriée avec l'ADN cible et avec l'oligonucléotide;

co-culture dans la cellule hôte microbienne appropriée à la fois de l'ADN cible et de l'oligonucléotide; et

maintien de ladite cellule hôte dans des conditions appropriées et pendant une période suffisante pour permettre l'incorporation dans l'ADN cible d'une troisième région complémentaire à la troisième portion entre la première région et la seconde région.

## Fig. 1

EP 0 253 193 B1

**Fig. 2**

Oligonucleotide

```
          5'
   CGGATCCCC A GGGAATTCAC
```

Linearized
Plasmid DNA

```
                  5'
 CGGATCCCC   GGGAATTCAC
 GCCTAGGGG   CCCTTAAGTG
            ↑
       Cleavage Site
```

**pWM300**

→

```
 CGGATCCCC A GGGAATTCAC
 CCCTAGGGG T CCCTTAAGTG
            ↑
        Mutation
```

**pWM301**

EP 0 253 193 B1

**Fig. 3**

5' CCAAGCTTGGCTCAGGTCGACGGATCCCC A GGGAATTCACTGGCCGTCGTTTTACAACGT 60

pWM300

**Fig. 4**

Fig. 5

Fig. 6

**Fig. 7**

**Fig. 8**

Distance: Mutation - Cleavage Site (nt)

**Fig. 9**

pWM300

5′———————————————A———————————————

5′···CCAAGCTTGGCTCAGGTCGACGGATCCCCGGGAATTCACTGGCCGTCGTTTTACAACGT···

HindIII  SalI  AccI  BamHI  XmaI  SmaI  EcoRI

**Fig. 10**

Efficiency of Mutagenesis (%) vs Distance from Cleavage Site (nt)

Mutant Colonies

EP 0 253 193 B1